# EUROPEAN PATENT APPLICATION

(11) **EP 3 906 919 A1**
(43) Date of publication of application: **10.11.2021**
(21) Application number: 20173318.5
(22) Date of filing: 06.05.2020
(51) Int. Cl.: A61K 31/122, A61K 31/194, A61K 31/4535, A61K 31/472, A61K 31/5415, A61K 31/55, A61K 31/225, A61K 31/4706, A61K 31/496

(54) **COMPOUNDS FOR THE TREATMENT OF COVID-19**

(71) Applicant: Dompe' Farmaceutici S.P.A., 20122 Milan (IT); Fraunhofer Gesellschaft zur Förderung der angewandten Forschung e.V., 80686 München (DE); Katholieke Universiteit Leuven KU Leuven Research & Development, 3000 Leuven (BE)
(72) Inventor: BECCARI, Andrea Rosario, 80131 Napoli (IT); MANELFI, Candida, 80131 Napoli (IT); TALARICO, Carmine, 80131 Napoli (IT); ZALIANI, Andrea, 22359 Hamburg (DE); GEISSLINGER, Gerd, 60596 Frankfurt am Main (DE); GRIBBON, Philip, 22587 Hamburg (DE)
(74) Representative: Mauri, Elisabetta Maria Ester

(57) **Abstract**

The present invention relates to compounds that are able to inhibt 3CL protease of COVID-19 virus, SARS-Cov-2

## Description

### FIELD OF THE INVENTION

The present invention relates to compounds that are able to inhibit the 3C-like protease (3CLP) of COVID-19 virus, SARS-CoV-2.

### STATE OF THE ART

Coronaviruses (Covs) are a large family of viruses belonging to the family Coronaviridae. The limited number of coronaviruses known to be circulating in humans were considered to cause mild infections and they were regarded in the past as relatively harmless respiratory human pathogens. However, in the last years the emergence of the severe acute respiratory syndrome coronavirus (SARS-CoV) and the Middle East Respiratory Syndrome (MERS) virus revealed that some coronaviruses can cause severe and sometimes fatal respiratory tract infections in humans (Pereira, H et al., 1989 Coronaviridae. Andrewes 'Viruses of Vertebrates, 5th ed.pp. 42-57; Holmes, K.V. et al., 1996. Virology 1,1075-1093). The first known case of SARS-CoV occurred in Foshan, China in November 2002 and new cases emerged in mainland China in February 2003. The first emergence of MERS-CoV occurred in June 2012 in Saudi Arabia. These events demonstrated that the threats of CoVs should not be underestimated and that it is of paramount importance to advance the knowledge on the replication of these viruses and their interactions with the hosts to develop treatments and vaccines.

In December 2019, atypical pneumonia cases emerged in China and the cause was identified as being a novel coronavirus named SARS-CoV-2 by WHO.

Investigations of the epidemiological and clinical characteristics, and outcomes of patients infected by SARS-CoV-2 demonstrated that the infection caused clusters of severe respiratory illness similar to the known SARS-CoV. Furthermore, it was demonstrated that the SARS-CoV-2 can cause severe illness in some patients, even initially it did not transmit readily between people. However, more recent epidemiological data suggest the new virus has undergone human host adaptation/evolution and has become more efficient in human-to-human transmission. Analysis of SARS-CoV-2 genome sequences obtained from patients during the beginning of the outbreak demonstrated that they are almost identical to each other and share 79.5% sequence identity to SARS-CoV. Furthermore, the SARS-CoV-2 is 96% identical at the whole genome level to a bat coronavirus. Phylogenetic analysis of CoVs of different species indicated that SARS-CoV-2 could have originated from Chinese horseshoe bats, but the intermediate transmission vehicle has not yet been identified (Dong, N. et al. Microbiology 2020). According to this study, SARS-CoV-2 belongs to a novel type of bat coronavirus owing to a high degree of variation from the human SARS virus. SARS-CoV-2 is the seventh member of the family of CoVs that infect humans.

Like SARS-CoV, SARS-CoV-2 enters target cells through an endosomal pathway and also uses the same cell entry receptor, Angiotensin-converting enzyme II (ACE2).

An effective therapeutic targets in SARS-CoV and several other CoVs, are replication-related enzymes, such as proteases (Zhou, P. et al. Microbiology 104, 2020). Drugs that inhibit conserved proteases are capable of preventing replication and proliferation of the virus by interfering with the post-translational processing of essential viral polypeptides. They can also reduce the risk of mutation-mediated drug resistance. This was the case for the SARS-CoV, as inhibitors targeting the main protease involved in replication and proliferation were the most effective means to alleviate the epidemic.

A particularly interesting therapeutic target is 3C-like proteinase (3CLP), a homodimeric cysteine protease and a member of a family of enzymes found in the Coronavirus polyprotein (Fan, K et al., J.Biol.Chem.279,1637-1642 (2004)). 3CLP in CoVs share conserved substrate recognition pockets, which are responsible for cleaving the viral polyprotein and the host factors involved in the innate immune response (Perlman, S et al., Nat. Rev. Microbiol. 7, 439-450). Due to its role in processing and virus maturation, 3CLP is considered to be a target for viral inhibitor development as an approach toward the severe acute respiratory syndrome coronavirus (SARS-Cov) treatment. (Ahn TY et al., Bull Korean Chem Soc. 2010;31 (1):87-91) (Liang PH et al., Curr. Top Med Chem. 2006;6(4):361-76) (Anand et al., Science 2003;300(5626):1763-7).

The identification and development of new drugs is a lengthy process that is not thus adequate to face the emergency of the immediate global challenge of COVID-19 outbreak.

Repurposing of drugs already tested as safe in man or approved for different therapeutic is a rapid response solution, since the pharmacokinetic, toxicological, and manufacturing data for the drigs are already available, thus allowing immediate application in clinical setting. 2006;6(4):361-76) (Anand et al., Science 2003;300(5626):1763-7).

Computational drug repurposing is an effective approach to find new identifications for already known drugs (Vanhaelen, Q et al., Drug Discov. Today 22, 210-222(2017) (Karman, B&Sippl, Curr.Med.Chem.26, 5389-5409(2019).

### SUMMARY OF THE INVENTION

The applicants have carried out the analysis of a library containing commercialized drugs and clinical candidates safe in man or characterized up to late clinical stage and have identified by Computer Associated Drug Design and in vitro screening a number of molecules able to bind to 3CLP and able to inhibit SARS-Cov-2 replication.

In details, the compounds identified as having inhibitory activity on replication of the virus are Proflavine hemisulfate, Raloxifen and Raloxin hydrochloride, Mequitazine, N-tert-Butylisoquine, Isofloxythepin, Succinobucol and Hypericin. All these compounds are well known approved drugs or drug candidates for different indications.

Proflavine Hemisulfate is the generic name of the hemisulfate salt of the compound 3,6-diaminoacridine having the following general formula (I):

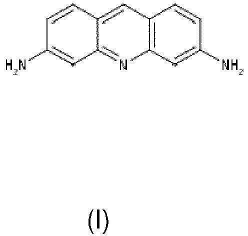

The compound is a topical antiseptic used mainly in wound dressings.

Raloxifen is the generic name of 1-[6-Hydroxy-2-(4-hydroxyphenyl)benzo[b]thien-3-yl]-1-[4-[2-(1-piperidinyl)ethoxy]phenyl]methanone, having the following general formula (II):

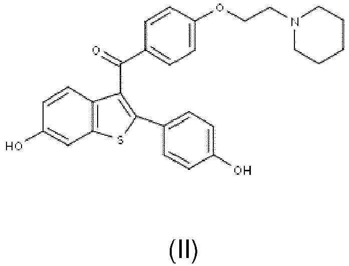

The compound is a selective benzothiophene estrogen receptor modulator (SERM) with lipid lowering effects and activity against osteoporosis and it is approved for the treatment and prevention of osteoporosis in postmenopausal women.

Mequitazine is the generic name of the compound 10-(Quinuclidin-3-ylmethyl)-10H-phenothiazine, having the following general formula (III)

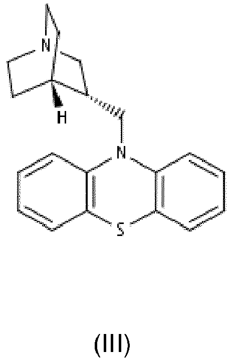

The compound is a histamine H1 receptor antagonist and it is approved for use in the treatment of allergic conjunctivitis, allergic rhinitis, pruritus and urticaria.

N-tert-Butylisoquine is the generic name of the compound 2-[(*tert*-butylamino)methyl]-5-[(7-chloroquinolin-4-yl)amino]phenol, an antimalarial drug, having the following general formula (IV):

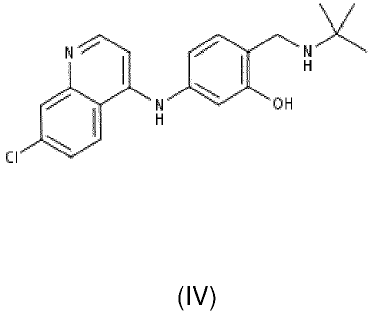

Isofloxythepin is the generic name of the compound 2-[4-(9-fluoro-3-propan-2-yl-5,6-dihydrobenzo[b][1]benzothiepin-5-yl)piperazin-1-yl]ethanol, having the following general formula (V):

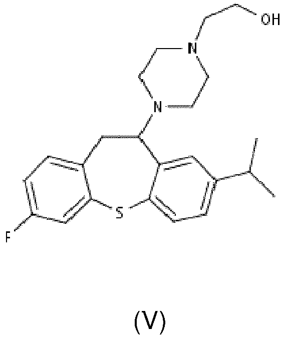

The compound is an antipsychotic agent.

Succinobucol is the generic name of the compound Succinic acid 2,6-di-tert-butyl-4-[1-(3,5-di-tert-butyl-4-hydroxyphenylsulfanyl)-1-methylethylsulfanyl]phenyl monoester (VI):

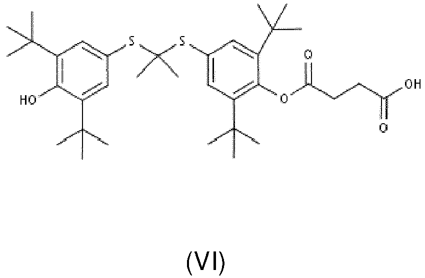

The compound is and antioxidant that was under clinical development for the treatment of atherosclerosis of the blood vessels of the heart, or coronary artery disease. Hypericin is the generic name of the anthraquinone derivative 1,3,4,6,8,13-Hexahydroxy-10,11-dimethylphenanthro[1,10,9,8-opqra]perylene-7,14-dione, having general formula (VII):

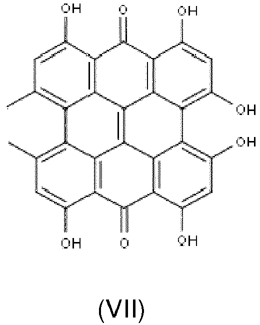

The compound is under development for the treatment cutaneous T-cell lymphoma. These compounds are therefore useful for the treatment of SARS-CoV-2 patients or for the prevention and/or treatment of contamination of a surface, including a body surface, or product with SARS-Cov-2.

Accordingly, a first object of the invention is a compound selected from Raloxifen and salts thereof, Mequitazine, N-tert-Butylisoquine, Isofloxythepin, Succinobucol and Hypericin for use in the treatment of humans who are infected with SARS-CoV-2, preferably for the treatment of COVID-19 patients.

A second object of the invention is a pharmaceutical composition comprising i) a compound according to the first object of the invention and ii) at least one inert pharmaceutically acceptable excipient, for use in the treatment of COVID-19.

A third object of the invention is a method of treatment of humans who are infected with SARS-CoV-2, preferably for the treatmnent of COVID-19 patients, comprising administering to a patient affected thereby a compound according to the first object of the invention.

A fourth object of the invention is the use of Proflavine hemisulfate for prevention and/or treatment of contamination of a surface with SARS-CoV-2. Preferably, said surface is a body surface.

### DETAILED DESCRIPTION OF THE INVENTION

A first object of the present invention is a compound selected from Raloxifen or a salt thereof, Mequitazine, N-tert-Butylisoquine, Isofloxythepin, Succinobucol and Hypericin for use in the treatment of humans who are infected with SARS-CoV-2, preferably wherein said humans have COVID-19.

Preferably, said salt of Raloxifen is Raloxifen hydrochloride.

As will be described in details in the experimental section, the present inventors have found that, in addition to their known activity, the above diverse compounds share the ability to bind the 3CLP of SARS-Cov-2 and thus to inhibit replication of the virus.

The selected compounds have the advantage that they have already been approved for clinical use or tested as safe in humans, in some cases are marketed drugs, and can therefore provide a rapid therapeutic approach to treatment of humans who are infected with SARS-CoV-2, preferably to the treatment of COVID-19 patients.

The compound according to the first object of the invention is administered in form of a pharmaceutical composition where the compound is admixed with one or more pharmaceutically acceptable excipients.

Thus, a second object of the invention is a pharmaceutical composition comprising the compound according to the first object of the invention and at least one inert pharmaceutically acceptable excipient, for use in the treatment of humans who are infected with SARS-CoV-2, preferably wherein said humans have COVID-19.

The route of administration of the compound or pharmaceutical composition of the present invention depends on the specific compound used or contained in the pharmaceutical composition and is in accordance with known methods for administration of the compound, which is usually by systemic administration, preferably by oral, parenteral or inhalatory route. The term parenteral as used herein includes intravenous, intraperitoneal, intracerebral, intrathecal, intracranial, intramuscular, intraarticular, intrasynovial, intrasternal, intraocular, intraarterial, subcutaneous, intracutaneous injection or infusion techniques.

The composition of the present invention may be formulated into oral, inhalatory or injectable dosage forms such as tablets, capsules,powders, solutions, suspensions, and emulsions.

Preferably, the pharmaceutically acceptable excipient in the pharmaceutical composition includes any and all solvents, diluents, or other vehicle, dispersion or suspension aids, surface active agents, isotonic agents, thickening or emulsifying agents, preservatives, solid binders, lubricants and the like, as suited to the particular dosage form desired. Some examples of materials which can serve as pharmaceutically acceptable excipient include, but are not limited to, sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatine; talc; cocoa butter and suppository waxes; oils such as peanut oil, cottonseed oi, safflower oil, sesame oil, olive oil, corn oil and soybean oil; glycols, such a propylene glycol; esters such as ethyl oleate and ethyl laurate; agar; buffering agents; alginic acid; pyrogen-free water; salts for regulating osmotic pressure; sterilized water; ethyl alcohol; preservatives, stabilizers, surfactants, emulsifiers, sweeteners, colorants, flavourings and the like.

The pharmaceutical composition of the present invention may be suitably formulated using appropriate methods known in the art or by the method disclosed in Remington's Pharmaceutical Science (recent edition), Mack Publishing Company, Easton Pa.

In one preferred embodiment, the invention provides for oral administration of the compound or the pharmaceutical composition of the invention

In such embodiment, the pharmaceutical composition is preferably in form of a tablet or capsule.

According to an alternative preferred embodiment, the compound or pharmaceutical composition of the invention is administered parenterally, preferably by intravenous administration or continuous infusion. This route of administration is particularly suitable for intubated or critically ill COVID-19 patients.

In yet an alternative preferred embodiment, the invention provides for direct administration of the compound or pharmaceutical composition of the present invention to the respiratory tract. The direct administration to the respiratory tract may be the sole route of administration of the compound or composition or may be combined with administration of the compound or composition via other systemic routes.

For direct administration of the compound or composition of the invention, a wide variety of devices that are designed for the direct delivery of pharmaceutical compositions and therapeutic formulations to the respiratory tract, may be used. In one aspect of the present invention, the compound or pharmaceutical composition of the present invention is administered in aerosolized or inhaled form.

The pharmaceutical composition of the present invention for direct administration to the respiratory tract as described above, can be in form of an aerosol formulation, as a dry powder or as a solution or suspension in a suitable diluent.

The amount of the compound according to the first object of the invention in the pharmaceutical composition of the present invention is the the amount usually employed for other indications of the specific compounds.

The amount can vary over a wide range depending on known factors, for example, the molecule used, the chosen route of administration and dosage form, the severity of the disease, the patient's age, body weight, general health status and sex, and the number of administrations per day. However, a person skilled in the art can determine the optimum amount in easily and routinely manner on the basis of the known dosage regime of the compound.

A third object of the invention is a method of treating humans who are infected with SARS-CoV-2, preferably COVID-19 patients, comprising administering to a patient affected thereby a compound selected from Raloxifen and salts therof, preferably the hydrochloride salt, Mequitazine, N-tert-Butylisoquine, Isofloxythepin, Succinobucol and Hypericin.

Among the compounds identified by the applicants as active against SARS-CoV-2 is Proflavine hemisulfate, which is a known antibacterial for topical use.

Accordingly, a fourth object of the invention is the use of Proflavine hemisulfate for prevention and/or treatment of contamination of a surface by SARS-CoV-2.

Preferably, said surface is a body surface.

The invention will be further described in the following examples, which do not limit the scope of the invention described in the claims.

### EXPERIMENTAL PART

### 1. Computer Associated Drug Design

The sequence and crystal structure of the 3C-like protease P of SARS-CoV-2, was obtained from the Protein Data Bank with the code 6LU7 (https://www.rcsb.org/structure/6lu7).

The EXSCALATE platform, the most powerful computing resources currently based in Europe to empower smart in-silico drug design (co-funded by the H2020-FET-HPC ANTAREX project), was exploited to perform molecular dynamics (MD) and docking simulations, with the final aim to select and make available molecules active against the 3C-like protease of SARS-CoV-2. Molecular dynamics simulations on the 3C-like protease of SARS-CoV-2 were performed to explore the conformational space of the active site of the protease, and select several protein conformations particularly suitable for docking simulations.

In the first step, MD simulations were carried out on the structure of the 3C-like protease of SARS-CoV-2, prepared ad hoc in order to optimize the 3D structure from a chemical and conformational point of view. The structure was firstly subjected to a cycle of energy minimization by steepest descent methods to eliminate all initial steric clashes and obtain a pre-equilibrated model to start from. Then a 100 ps restrained MD simulation (typically 1000 KJ/mole force constant) was performed on the solvent atoms to equilibrate water molecules keeping the solute restrained. Finally, a production run was performed to generate a 1microsecond trajectory with a total of 20.000 collected. Post HPC-run analysis of the results was performed.

In a second step a High Performance Computing (HPC) simulation was conducted to virtual screen the Safe in Man (SIM) library, containing commercialized and under development drugs, already proved safe in man (> 10.000 drugs), against the 3C-like protease of SARS-CoV-2, on both the crystal structures and the most 100 relevant conformations extracted from MD runs.

The SIM library is a Dompé dataset containing about 10.000 pharmaceutical compounds, built merging the list of drugs launched or under active development in several clinical phases with molecules in early phases (biological testing and preclinical) matching the query "CoV Inhibitors" as mechanism of action. Both lists derived from an Integrity database search (https://clarivate.com/cortellis/solutions/pre-clinical-intelligence-analytics/). All the compounds have been classified based on the mechanism of action of the class (antibacterial, antiviral, antiparasitic, antifungal, etc...) and on merging information reported in the Integrity database with those included in the drug repositioning database "RepoDB". Also drug information provided by DrugCentral, related to pharmaceutical products, drug mode of action, indications and pharmacologic action, was integrated in Dompé drug virtual library, as well as information on drugs and drug targets reported in the DrugBank database.

The simulation was performed using LiGen™ (Ligand Generator), the de novo structure based virtual screening software, designed and developed to run on HPC architectures, which represent the most relevant tool of the EXSCALATE platform. LiGenTM is formed by a set of tools that can be combined in a user-defined manner to generate project centric workflows. In particular, LiGenDock is a docking module using LiGenScore to compute the scoring function and the LiGenPass and LiGenPocket modules to obtain the 3D structure of the binding site. Both the docking algorithm implemented in LiGen, the pharmacophoric docking (LiGenPh4) and the geometrical docking (LiGenGeodock), as well as the different scoring functions calculated, were used in this study to explore different protocols of Virtual Screening (VS) and select the best one in terms of performance.

Usually, the performance of a VS protocol is assessed by evaluating its capacity to recognize the active molecules among a large number of inactive decoys, where usually the active molecules represent the 1% of the total number of compounds. The performances of the tested VS strategy was assessed by evaluating the capacity to correctly rank molecules which are endowed with antiviral activity, and in particular with a known effect against coronaviruses. Following this approach, about 130 molecules were labelled as active compounds.

The VS protocol performance was thus evaluated comparing the screening results of the SIM library on the crystal structure of 3C-like protease of SARS-CoV-2 and on the most 100 relevant conformations extracted from MD runs, by using different docking algorithms and different scoring function. The conditions showing the best capacity to recognize the active molecules were used to prioritize the total list of screened molecules and select the top scored compounds, according to the score value (Csopt), that predicts the binding affinity of the molecules in the protein binding site. The molecules Proflavine hemisulfate, Raloxifen, Mequitazine, N-tert-Butylisoquine, Isofloxythepin, Succinobucol and Hypericin showed a Csopt value higher than 6.3 and were selected for further validation of the inhibitory activity in in vitro experiments.

### 2. In vitro screening of activity against SARS-CoV-2

The compounds selected by the CADD analysis were tested in a SARS-CoV 2 VeroE6-EGFP HTS antiviral Assay. This assay is based on viral infection of VeroE6 cells followed by visual monitororing of cytopathic effects.

In details, the EGFP-fluorescent-reporter constitutively expressing cells line VeroE6-EGFP was received from JNJ whereas the SARS-CoV-2 was received from a Belgian strain (BetaCov/Belgium/GHB-03021/2020).

The test compounds were dissolved at 10mM in dimethylsulphoxide (DMSO) and then diluted in cell culture medium to a final DMSO concentration below 0.5 % and mixed with 30 CCID50 of SARS-CoV and 2000 VeroE6-EGFP cells per well in 384-well plates. As a control, wells containg 30 CCID50 of SARS-CoV and 2000 VeroE6-EGFP cells per well were also set up.

After incubation at 37°C for 5 days the EGFP signal of each well was recorded using an ArrayScan, which is a LED-based high-content imager

Standard whole-well fluorescence plate readout was performed (self-optimizing protocol, ∼6min per 384-well plate, 4 reads/well) and the fluorescence total intensity of the test compounds wells and of the control wells was measured.

High-content imaging readout was also performed using a 5x objective, allowing to capture almost the entire well of a 384-well plate at once (auto-focus on each well, no binning, 1 channel). Two values were obtained from high content imaging:
**% Confluence** is the percentage increase in confluence in the test compound wells compared to the control wells, based on readout "SpotTotalAreaCh2" which is the total surface of the field of view that is green. Since the GFP marker is located in both the cell cytoplasm and in the nucleus, it allows to calculate the surface of the well that is (still) covered by cells (a large value means that there are still a lot of cells on the microtiter plate bottom surface, a small value means that most of the fluorescence i.e. the cells are gone).
**% Inhibition** is the percentage increase in the number of cells in the test compound wells compared to the control wells, based on "ValidObjectCount" which is the total count of the number of nuclei. The nuclei are brighter green than the cytoplasm, which allows to count the number of cells, providing that the cell density is not too high.

The results obtained for wach of the compounds tested are shown in Table 1 below.

**Table 1**

| Compound | **% Confluence** | **% Inhibition** |
|---|---|---|
| Proflavine hemisulfate | 8.56 | 208.4 |
| Raloxifen hydrochloride | 45.26 | 70.75 |
| Mequitazine | 61.74 | 62.09 |
| N-tert-Butylisoquine | 77,86 | 44,99 |
| Isofloxythepin | 67,67 | 35,39 |
| Succinobucol | 18,73 | 37,53 |
| Hypericin | 20.85 | NA |

As can be see from the results, all compounds are able to inhibit virus replication and consequent cytopathic effects of the virus compared to the control.

## Claims

1. A compound selected from Raloxifen or a salt thereof, Mequitazine, N-tert-Butylisoquine, Isofloxythepin, Succinobucol and Hypericin for use in the treatment of humans who are infected with SARS-CoV-2.

2. A compound as claimed in claim 1, wherein said salt of Raloxifen is Raloxifen hydrochloride.

3. A pharmaceutical composition comprising:
i) a compound selected from Raloxifen or a salt thereof, Mequitazine, N-tert-Butylisoquine, Isofloxythepin, Succinobucol and Hypericin for use in the treatment of humans who are infected with SARS-CoV-2ii) at least one inert pharmaceutically acceptable excipient,
for use in the treatment of COVID-19.

4. A compound for use according to claim 1 or 2 or a pharmaceutical composition for use according to claim 3, wherein said compound or composition is administered orally.

5. A pharmaceutical composition for use according to claim 4, in form of a table or capsule.

6. A compound for use according to claim 1 or 2 or a pharmaceutical composition for use according to claim 3, wherein said compound or composition is administered parenterally.

7. A compound for use or pharmaceutical composition or use as claimed in claim 7, wherein compound or composition is administered by intraveneous administration out continuous infusion.

8. Use of Proflavine hemisulfate for prevention and/or treatment of contamination of a surface with SARS-CoV-2.

9. Use as claimed in claim 8, wherein said surface is a body surface.
